# EUROPÄISCHE PATENTSCHRIFT

(11) **EP 2 066 333 B1**
(45) Veröffentlichungstag und Bekanntmachung des Hinweises auf die Patenterteilung: **25.12.2013**
(21) Anmeldenummer: 07820552.3
(22) Anmeldetag: 25.09.2007
(51) Int. Cl.: A61K 36/84, A61P 25/20

(54) **BALDRIAN-EXTRAKTZUBEREITUNG**
VALERIAN EXTRACT PREPARATION
PRÉPARATION D'UN EXTRAIT DE VALÉRIANE

(30) Priorität: 27.09.2006 DE 102006045974
(43) Veröffentlichungstag der Anmeldung: 10.06.2009
(73) Patentinhaber: Finzelberg GmbH & Co. KG, 56626 Andernach (DE)
(72) Erfinder: FEISTEL, Björn, 56626 Andernach (DE); SIEVERS, Hartwig, 91413 Neustadt (DE); LEHNFELD, Romanus, 91074 Herzogenaurach (DE)
(74) Vertreter: von Kreisler Selting Werner
(86) Internationale Anmeldenummer: PCT/EP2007/060153
(87) Internationale Veröffentlichungsnummer: WO 2008/037707

(56) Entgegenhaltungen:
- EP-A- 0 363 777
- HATTESOHL M ET AL: "Effects of extracts from Valeriana officinalis L. in pharmacological studies" PLANTA MEDICA, Bd. 72, Nr. 11, September 2006 (2006-09), Seite 1064, XP009101983 & 54TH ANNUAL CONGRESS ON MEDICINAL PLANT RESEARCH; HELSINKI, FINLAND; AUGUST 29 -SEPTEMBER 02, 2006 ISSN: 0032-0943
- MATSUNAGA KIMIHIRO ET AL: "Effects of Chinese and Paraguayan medicinal plants on the duration of immobility of mice in the forced swimming test" NATURAL MEDICINES, Bd. 51, Nr. 1, 1997, Seiten 63-66, XP009082324 ISSN: 1340-3443
- SAKAMOTO T ET AL: "PSYCHOTROPIC EFFECTS OF JAPANESE VALERIAN ROOT EXTRACT" CHEMICAL AND PHARMACEUTICAL BULLETIN (TOKYO), Bd. 40, Nr. 3, 1992, Seiten 758-761, XP009101942 ISSN: 0009-2363
- IZQUIERDO SANCHEZ T ET AL: "The effect of valerian (valerian officinalis L.) in growth inhibition and its anxiolytic activity in mice" REVISTA MEXICANA DE CIENCIAS FARMACEUTICAS 2001 MX, Bd. 32, Nr. 4, 2001, Seiten 24-28, XP001538185 ISSN: 1027-3956
- HATTESOHL MIGUEL ET AL: "Extracts of Valeriana officinalis L. sl show anxiolytic and antidepressant effects but neither sedative nor myorelaxant properties" PHYTOMEDICINE (JENA), Bd. 15, Nr. 1-2, Januar 2008 (2008-01), Seiten 2-15, XP002485269 ISSN: 0944-7113
- DATABASE WPI Week 200278 Thomson Scientific, London, GB; AN 2002-714298 XP002493025 & CN 1 191 137 A (HUBEI INST GERIATRICS) 26. August 1998 (1998-08-26)
- DATABASE WPI Week 199751 Thomson Scientific, London, GB; AN 1997-550666 XP002493026 & CN 1 129 567 A (YAO L) 28. August 1996 (1996-08-28)

## Beschreibung

Die vorliegende Erfindung betrifft eine neue Baldrian-Extraktzubereitung, ein Verfahren zu seiner Herstellung und seine Verwendung.

Die Baldrianwurzel (Radix Valeriana officinalis) wird schon lange bei Unruhezuständen sowie nervösbedingten Einschlafstörungen eingesetzt. Trotz zahlreicher Untersuchungen ist nicht vollständig geklärt, welche Inhaltsstoffe für die Wirkung verantwortlich sind.

WO 98/13054 beschreibt, dass Valepotriate sedative Eigenschaften aufweisen. Da die Valepotriate jedoch in wässrigen und wässrig-alkoholischen Lösungsmitteln praktisch unlöslich sind, sind sie in wässrigen oder alkoholischen Extrakten von Radix valeriana praktisch nicht nachweisbar.

Ein weiterer Inhaltsstoff, dem die Wirksamkeit teilweise zugeschrieben wird, sind die Valerensäuren, Sesquiterpenverbindungen. Typische Baldrian-Extrakte werden auf den Gehalt an Valerensäuren standardisiert.

Die WO 98/13054 beschreibt Extrakte, die mit CO₂-Extraktion erhalten werden und dadurch hohe Gehalte sowohl an Valerensäure als auch von Valepotriaten aufweisen.

US 6,383,526 beschreibt ein Verfahren zur Herstellung eines Baldrian-Extraktes, bei dem die Extraktion mit 50 bis 100% (v/v) Ethanol erfolgt (typischerweise 70%). Hierdurch werden die Gehalte an Valepotriaten reduziert, jedoch Extrakte erhalten, die hohe Gehalte an Valerensäuren aufweisen. Für diese Extrakte ist eine anxiolytische Wirkung beschrieben.

Aufgabe der vorliegenden Erfindung war es, Baldrian-Extraktzubereitung bereitzustellen, die verbesserte Eigenschaften aufweisen, insbesondere eine verstärkte anxiolytische oder antidepressive Wirkung zeigen.

Überraschenderweise wurde gefunden, dass die Aufgabe gelöst werden kann durch ein Verfahren zur Herstellung einer Baldrian-Extraktzubereitung umfassend die Schritte der
a) Extraktion von Radix Valeriana mit alkoholisch-wässrigen Extraktionsmitteln, um einen Rohextrakt zu erhalten, wobei das alkoholisch-wässrige Extraktionsmittel zwischen 10 und 50 Vol.-% Alkohol enthält;
b) zumindest teilweises Entfernen des alkoholischen Anteils aus dem Rohextrakt, um einen Dickextrakt zu erhalten;
c) Inkontaktbringen des Dickextraktes mit einem hydrophoben Adsorptionsmittel;
d) Abtrennen des hydrophoben Adsorptionsmittel, um einen gereinigten Extrakt zu erhalten;
e) Überführung des gereinigten Extraktes in eine Baldrian-Extraktzubereitung mit pharmazeutisch üblichen Hilfsstoffen.

Bei dem erfindungsgemäßen Verfahren wird also zunächst Radix Valeriana officinalis mit einem alkoholisch-wässrigen Extraktionsmittel extrahiert. Die Droge wird hierzu typischerweise zerkleinert (8-12 mm), um die Extraktion zu erleichtern. Ein geeignetes Gewichtsverhältnis zwischen Droge und Extraktionsmittel liegt im Bereich von etwa 1:5 bis 1:30, bevorzugt 1:10 bis 1:20.

Dem Fachmann ist bekannt, dass die Extraktion durch ein Erwärmen des Extraktionsmittels verbessert werden kann. Geeignete Temperaturen für die Extraktion liegen zwischen Raumtemperatur und etwa 70°C, bevorzugt im Bereich von 40 bis 60°C.

Je länger extrahiert wird, desto mehr Inhaltsstoffe können extrahiert werden. Auf der anderen Seite können Extraktinhaltsstoffe durch die Temperaturbehandlung bei der Extraktion auch beschädigt werden. Typische Extraktionszeiten liegen zwischen 1 und 12 Stunden, bevorzugt 2 bis 6 Stunden.

Im Gegensatz zu den im Stand der Technik beschriebenen Verfahren wird die Extraktion mit einem Extraktionsmittel durchgeführt, das einen eher geringen Anteil an Alkohol enthält. Der Gehalt an Alkohol liegt zwischen 10 und 50 Vol.-% des Extraktionsmittels, bevorzugt zwischen 20 und 45 Vol.-%, noch mehr bevorzugt zwischen 30 und 40 Vol.-%. Geeignete Alkohole sind insbesondere Ethanol, aber auch Methanol, Isopropanol und Mischungen hiervon.

Nach der Extraktion wird das Extraktionsmittel vom Drogenrückstand getrennt. Geeignete Verfahren hierfür sind Filtration, Absaugen, Ablassen, etc.

Anschließend wird der alkoholische Anteil des so erhaltenen Rohextraktes ganz oder teilweise entfernt. Dies kann beispielsweise mit einem Blasenverdampfer oder mit einem Plattenverdampfer erfolgen. Durch Zugabe von weiterem Wasser kann der Alkoholanteil reduziert werden, beispielsweise < 2 Gew.-%. Der resultierende Trockensubstanzanteil liegt bei 40 bis 70% (m/m).

Der so erhaltene wässrige Extrakt wird im folgenden als Dickextrakt bezeichnet.

Im nächsten Schritt wird der Dickextrakt mit einem hydrophoben Adsorptionsmittel in Kontakt gebracht. Es ist wünschenswert, dass der Adsorber eine große Oberfläche aufweist (> 200 m²/g), um eine große Menge an Substanz adsorbieren zu können. Besonders vorteilhaft erweisen sich Harze mit gleichmässigen Porengrößen (100 bis 450 Å). Als besonders geeignet haben sich Kunstharzadsorber erwiesen, beispielsweise auf Basis von Divinylbenzolcopolymeren, aliphatischen Esterpolymeren oder Formophenolpolymeren. Bevorzugt weisen die eingesetzten Adsorber keine funktionellen Gruppen wie beispielsweise quartäre Ammoniumverbindungen oder Säurefunktionalitäten auf, wie sie bei Ionenaustauschern üblich sind. Besonders geeignete Substanzen sind unter dem Handelsnamen Amberlite^{®} erhältlich. Geeignete Produkte sind XAD4, XAD2, XAD16, XAD761, XAD1180, XAD160, XAD7HP. Verwendet werden können auch analoge charakterisierte Adsorberharze, z.B. von der Firma Diaion, von der Firma Bayer (Lewatit^{®}) oder der Firma Miontech.

Das Inkontaktbringen des Dickextraktes mit dem hydrophoben Adsorptionsmittel kann mit dem Fachmann geläufigen Verfahrensarten durchgeführt werden, beispielsweise, in dem das Adsorbermaterial in eine Säule gefüllt wird, durch das der Dickextrakt (gegebenenfalls nach Verdünnung) durchfließt. Eine Alternative hierzu ist ein Batchverfahren, bei dem das Adsorptionsmittel zugegeben wird und nach einer Verweilzeit wieder abgetrennt wird.

Nach dem Abtrennen des hydrophoben Adsorptionsmittels wird ein Baldrian-Extrakt erhalten, der in dem Fachmann bekannter Weise weiterbehandelt werden kann. Bevorzugt wird der Extrakt getrocknet. Dies kann beispielsweise erfolgen durch Lyophilisieren, Sprühtrocknen, Vakuumtrocknen, etc. Bevorzugt werden beim Sprühtrocknen und Vakuumtrocknen Hilfsstoffe zugesetzt, um einen rieselfähigen Trockenextrakt zu erhalten.

Gegenstand der Erfindung ist auch eine Baldrian-Extraktzubereitung, die nach dem erfindungsgemäßen Verfahren erhältlich ist.

Überraschenderweise zeigt die erfindungsgemäße Baldrian-Extraktzubereitung einen relativ geringen Gehalt an Gesamt-Valerensäuren (≤ 0,18 Gew.-%) sowie einen relativ kleinen Gehalt an Valepotriaten (≤ 0,1 Gew.-%), jeweils bezogen auf den Trockenextrakt.

Typischerweise verteilen sich die Gehalte an Gesamt-Valerensäuren wie folgt:
- < 0,10 Gew.-% Valerensäure,
- < 0,1, mehr bevorzugt < 0,07, noch mehr bevorzugt < 0,05 Gew.-% Acetoxyvalerensäure,
- ≤ 0,01, bevorzugt < 0,01 Gew.-% Hydroxyvalerensäure
jeweils bezogen auf den Trockenextrakt.

Soweit nicht anders angegeben, sind alle %-Angaben "Gew.-%".

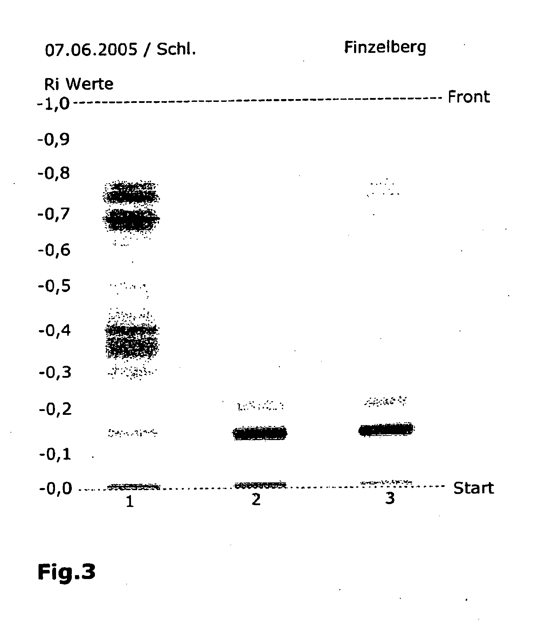

Durch das Verfahren erfolgt eine Abreicherung von besonders lipophilen Inhaltsstoffen charakterisiert durch ein Fehlen oder eine stark verminderte Konzentration im R_{f}-Bereich von 0,3 bis 0,8 im Vergleich zum Ausgangsextrakt (siehe Figur 3). Die dünnschichtchromatographischen Bedingungen (gemäß Figur 3) sind wie folgt:
Stationäre Phase (DC-Plattenmaterial): Kieselgel 60 F₂₅₄
Mobile Phase (Laufmittel): s. Fließmittel

| | |
|---|---|
| Fließmittel | Dichlorethan : Essigsäure : Methanol : Wasser 50 : 25 : 15 : 10 (V/V/V/V) |
| | (mit ca. 30 min. Kammersättigung) |
| Trennstrecke | 15 cm (Start bis Front) |
| Laufzeit | Ca. 2 Stunden |
| Trocknung | Ca. 10 min. im Kaltluftstrom |
| Sprühreagenz | Anisaldehyd - Reagenz |
| | 0,5 mL Anisaldehyd werden mit 10 mL Essigsäure wasserfrei.,85 mL Methanol und 5 mL Schwefelsäure 96% in der angegebenen Reihenfolge gemischt. |
| Trocknung | Ca. 3 min. bei 120°C bis zur optimalen Farbentwicklung der Zonen. |
| Probenlösung | 1g Baldrianextrakt wird mit 10 mL Ethanol 50% V/V 10 min lang im Wasserbad bei 65°C gelöst. Nach dem Abkühlen auf Raumtemperatur wird klar filtriert und das klare Filtrat zur Chromatographie verwendet. |
| | Es werden 10 µl mit Auftragegerät aufgebracht. |

Die erfindungsgemäß Extraktzubereitung weist bevorzugt ein Drogen/Extrakt/Verhältnis zwischen 3:1 und 10:1, bevorzugt zwischen 4:1 und 6:1 auf.

Gegenstand der Erfindung ist auch ein Arzneimittel, das die erfindungsgemäße Baldrian-Extraktzubereitung enthält sowie die Verwendung der erfindungsgemäßen Baldrian-Extraktzubereitung zur Herstellung eines Arzneimittels zur Angsthemmung oder Angstlösung (Anxiolytikum) und zur Minderung von Depressionen oder depressiven Stimmungen (Antidepressivum).

Entscheidend ist, dass die anxiolytische Eigenschaft und die antidepressive Eigenschaft nicht durch eine sedative Wirkung erreicht werden, sondern wie in den Beispielen 7 und 8 gezeigt wird, keine sedative Wirkung vorliegt.

Der erhaltene Extrakt kann in einfacher Weise in eine pharmazeutische Zubereitung überführt werden, beispielsweise in Form von Tabletten, Kapseln, Pastillen.

Als geeignet haben sich insbesondere Dosierungen von etwa 100 bis 1.000 mg pro Dosiseinheit erwiesen, wobei pro Tag bevorzugt etwa 1 bis 3 Dosiseinheiten einzunehmen sind.
Figur 1a/b zeigt einen EPM Test bei Verabreichung des erfindungsgemäß aufgereinigten Baldrianextraktes.
Figur 2a/b zeigt einen EPM Test der Rückstandsfraktion der Reinigung.
Figur 3 zeigt eine dünnschichtchromatographische Analyse der erhaltenen Fraktionen.

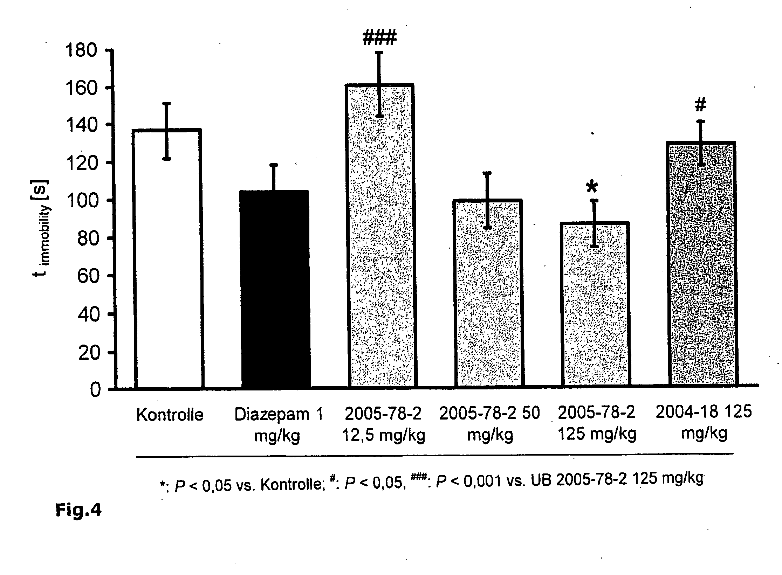

Figur 4 zeigt einen Forced Swimming Test des erfindungsgemäßen Extraktes im Vergleich zu einem nicht aufgereinigten Extrakt nach 16-tägiger, 2 x täglicher peroraler Gabe.

Die Erfindung wird durch die nachfolgenden Beispiele näher erläutert.

### Beispiel 1: Herstellung des Dickextraktes

10 kg von Radix Valerianae offic. (Valerensäuren 0,18%) wurden mit 35% v/v Ethanol im Verhältnis 1:16 versetzt und bei 50°C im Holstein-Kappert-Extrakteur erschöpfend perkoliert. Das Eluat wurde von der Droge abgelassen und über einen 250 µm Siebbeutel-Durchlass von Drogenresten befreit. Das Perkolat wurde in einem Plattenverdampfer eingedampft und durch Wasserzugabe lösemittelfrei gefahren. Als Ausbeute resultierten 4,6 kg Dickextrakt mit einem Trockensubstanzanteil von 60,3%.

### Beispiel 2a): Reinigung durch hydrophoben Absorber XAD-4

Hiervon wurde ein Anteil von 663,3 g Dickextrakt, entsprechend 400 g Trockenextraktequivalent mit demineralisiertem Wasser auf 10% Trockensubstanzanteil verdünnt und 30 Minuten lang unter Rühren homogenisiert. Es resultierte eine dunkle, homogene Lösung.

Diese wurde auf ein hydrophobes Adsorberharz (Amberlite XAD-4) in einer Glassäule gegeben, die mit ca. 4 l feuchtem Harz, entsprechend ca. 1600 g Trockenharz, gefüllt ist. Mit einer Geschwindigkeit von 3 Bettvolumina der Harzes pro Stunde erfolgte der Reinigungsschritt.

Nach Reinigung der Ausgangsextraktlösung konnten noch 85% der Ausgangsmenge festgestellt werden. Diese Lösung wurde beigedampft und mit dem Trockenhilfsstoff Maltodextrin im Verhältnis 70% nativ : 30% Maltodextrin per Sprühtrocknung in eine Trockenextraktzubereitung überführt (UB 2005-37-2).

### Beispiel 2b): Reinigung durch hydrophoben Adsorber XAD-1180

Basierend auf einem Baldrian-Dickextrakt gemäß Beispiel 1 wurde ein Anteil von 2,7 kg Extrakt, entsprechend 1628 g Trockenextraktequivalent mit demineralisiertem Wasser auf 10% Trockensubstanzanteil verdünnt und 30 Minuten lang unter Rühren homogenisiert. Es resultierte eine dunkle, homogene Lösung.

Diese wurde auf ein hydrophobes Adsorberharz (XAD-1180) in eine Säule gegeben, die mit ca. 20 l feuchtem Harz, entsprechend ca. 8000 g Trockenharz, gefüllt ist. Mit einer Geschwindigkeit von 3 Bettvolumina der Harzes pro Stunde erfolgte der Reinigungsschritt. Nach Reinigung der Ausgangsextraktlösung konnten noch 81% der Ausgangsmenge festgestellt werden.

Diese Lösung wurde zum Dickextrakt beigedampft und mit dem Trockenhilfsstoff Maltodextrin im Verhältnis 70% nativ : 30% Maltodextrin per Sprühtrocknung in eine Trockenextraktzubereitung überführt (UB 2005-78-2). Diese Baldrianextraktzubereitung hatte einen Trocknungsverlust von 4,6% und enthielt: < 0,02% Valepotriate, 0,112% Gesamt-Valerensäuren, hiervon 0,06% Valerensäure, 0,04% Acetoxyvalerensäure und 0,002 % Hydroxyvalerensäure.

### Beispiel 3: Rückstand des Adsorbers

Die vom Adsorber in Beispiel 2a zurückgehaltenen Substanzen wurden durch Elution mit 2 Bettvolumina an Ethanol 96% V/V eluiert. Auch diese Lösung wurde zum Dickextrakt beigedampft und mit dem Trockenhilfsstoff Maltodextrin im Verhältnis 70% nativ : 30% Maltodextrin per Sprühtrocknung in eine Trockenextraktzubereitung überführt (UB 2005-37-1).

### Beispiel 4: Gehalt an Valerensäuren

Anschließend wurden die Valerensäuren der Extrakte gemäß Beispiele 2a) und 3 bestimmt.

| | Lipophile Ehanolphase von Adsorbereinschluss (Beispiel 3) | Gereinigte Wasserphase nach Adsorberkontakt (Beispiel 2a)) |
|---|---|---|
| Gesamt-Valerensäuren im Trockenextrakt | 0,73% | 0,13% |

### Beispiel 5: Vergleichsbeispiel ohne Reinigung

Basierend auf dem Baldrian-Dickextrakt gemäß Beispiel 1 wurde mit dem Trockenhilfsstoff Maltodextrin im Verhältnis 70% nativ : 30% Maltodextrin per Sprühtrocknung eine Trockenextraktzubereitung hergestellt (UB 2004-18). Diese Baldrianextraktzubereitung hatte einen Trocknungsverlust von 3,0% und enthielt: 0,04% Valepotriate, 0,29% Gesamt-Valerensäuren, hiervon 0,12% Valerensäure, 0,14% Acetoxyvalerensäure und 0,03 % Hydroxyvalerensäure.

### Beispiel 6: Anxiolytische Wirkung

Das Elevated Plus Maze (EPM) ist ein weit verbreitetes und anerkanntes Verhaltensmodell, das geeignet ist, das Angstverhalten von Ratten bzw. Mäusen zu untersuchen und die Detektion anxiolytisch oder anxiogen wirkender Substanzen erlaubt. Es basiert auf der Beobachtung, dass unkonditionierte Ratten oder Mäuse auf natürliche aversive Stimuli mit spontanem Vermeidungsverhalten reagieren.

Basierend auf Untersuchungen Montgomerys stellte Pellow 1985 das EPM erstmals vor (Pellow, S., et al., 1985. Validation of open:closed arm entries in an elevated plus-maze as a measure of anxiety in the rat, J Neurosci Methods 14, 149-167).

Beim EPM handelt es sich um eine erhöht aufgestellte plusförmige Versuchsapparatur, an deren quadratischer zentraler Plattform sich jeweils zwei geschlossene und zwei offene Stege gegenüberstehen. Die geschlossenen "Arme" weisen eine hohe Umrandung auf, während die offenen Stege von einer schmalen Leiste umgeben sein können. Dabei stellen die geschlossenen Arme einen geschützten Bereich dar, der dem Versuchstier die Möglichkeit der Deckung gewährt, während die offenen Stege aversiv wirken.

Tiere, die über einen definierten Zeitraum alle Bereiche des Maze frei erkunden können, meiden die offenen Arme und bevorzugen die geschlossenen Bereiche, was sich in einer geringeren Aufenthaltsdauer auf den offenen Stegen und in einer verminderten Anzahl an Eintritten auf diese ausdrückt. Das durch die Angst bedingte Vermeidungsverhalten überwiegt der Neugier, eine neue Umgebung zu erkunden ("Exploratory Behavior").

Während einer Testperiode werden die Zahl der Eintritte auf die offenen und in die geschlossenen Arme sowie die Zeit auf offenen und in den geschlossenen Armen erfasst. Beide Parameter werden durch anxiolytisch wirkende Substanzen gesteigert, durch Anxiogene vermindert.

Falsch positive Ergebnisse können nur durch motilitätssteigernde Substanzen verursacht werden, sie werden dadurch eliminiert, dass nicht die Absolutwerte, sondern die jeweiligen Prozentzahlen bewertet werden (Hogg, S., 1996. A review of the validity and variability of the elevated plus-maze as an animal model of anxiety, Pharmacol Biochem Behav 54, 21-30).

Das EPM erlaubt eine zuverlässige Detektion von Benzodiazepinen (z.B. Diazepam^{®}). Da eine Vielzahl an Einflussfaktoren (Licht, akustische Reize) das Modell beeinflussen, muss eine Validierung des Testsystems vorhergehen.

Jeder Prüfsubstanz ist immer eine Referenzsubstanz beizustellen. Bei entsprechender Handhabung und Auswertung werden zuverlässig anxiolytische und anxiogene Effekte angezeigt. Falsch positive Ergebnisse sind hiernach nahezu ausgeschlossen.

Die Testungen der Valeriana-Zubereitungen im EPM erfolgten mit weiblichen NMRI-Mäusen (n = 10-14) unter Verwendung folgender Versuchsanordnung:

| Tierstamm | Geschlecht | Testerfahrung | Nahrung | Leisten (offene Arme) | Farbe Steguntergrund |
|---|---|---|---|---|---|
| NMRI | weiblich | naiv | nicht depriviert | ohne Leisten | schwarz |

### Referenzextrakte

Es ergibt sich folgender Befund für verschiedene Extrakte, die aus einer Drogencharge gewonnen wurden:

| Auszugsmittel | Wasser | Vergleichsextrakt EtOH 35% V/V | Vergleichsextrakt EtOH 70% V/V | Überkritisches Kohlendioxid |
|---|---|---|---|---|
| DEV nativ | 3,3 : 1 | 3,1 : 1 | 4,0 : 1 | 40 : 1 |
| Gehalt Gesamt-Valerensäuren | 0,05 % | 0,25 % | 0, 80 % | 12,70 % |
| Gehalt Valepotriate | < 0,02 % | < 0,02 % | < 0,02 % | 11,6 % |
| Anxiolytische Eigenschaften im EPM-Modell (weibl. NMRI-Mäuse nüchtern, n = 10, 60 min. nach oraler Gabe), 250 mq/kq KG | | | | |
| %-Anteil Verweildauer offene Arme | + 10 % | + 24 % | + 17 % | + 11 % |
| %-Anteil Eintritte offene Arme | + 6 % | + 18 % | + 8 % | - 3 % |
| Wertung | Nicht signifikant | Signifikant gegenüber Kontrollgruppe | Nicht signifikant | Nicht signifikant |

### Erfindungsgemäße Extrakte

Erfindungsgemäßer Extrakt, Charge UB 2007-37-2 gemäß Beispiel 2a)

| | |
|---|---|
| Auszugsmittel | EtOH 35% V/V |
| Aufreinigung | Durchlauf Adsorbersäule mit XAD-4 Harz |
| DEV nativ | 4,2 : 1 |
| Gehalt Gesamt-Valerensäuren | 0,13 % |
| Gehalt Valepotriate | < 0,01 % |

| Anxiolytische Eigenschaften im EPM-Modell (weibl. NMRI-Mäuse nüchtern, n = 10, 60 min. nach oraler Gabe), 250 mq/kq KG | |
|---|---|
| %-Anteil Verweildauer offene Arme | + 16 % |
| %-Anteil Eintritte offene Arme | + 17 % |
| Wertung | Hoch Signifikant gegenüber Kontrollgruppe |

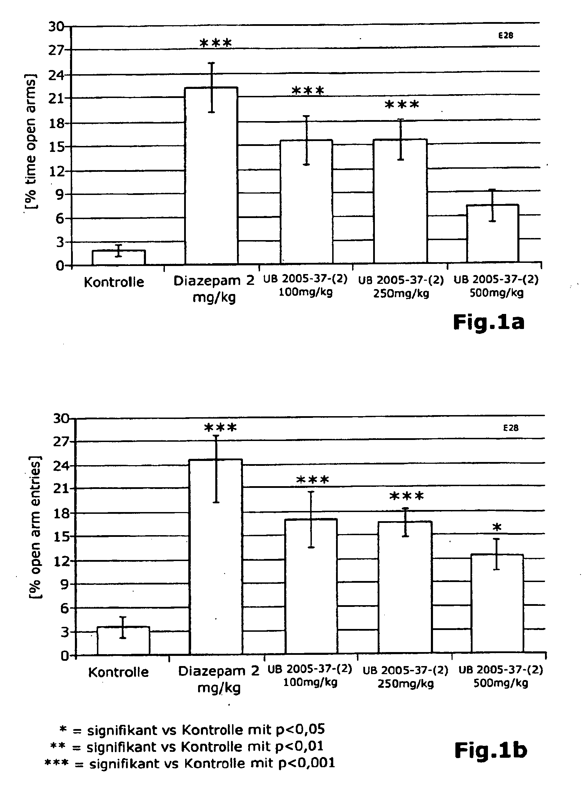

Figur 1a/b zeigen den EPM Test bei Verabreichung des erfindungsgemäß aufgereinigten Baldrian-Extraktes gemäß Beispiel 2a).

Figur 1a) zeigt die Zeit, die auf offenen Armen verbracht wird.

Figur 1b) zeigt die Anzahl der Eintritte in die offenen Arme.

Im Vergleich zur Kontrolle zeigt sich eine deutliche anxiolytische Wirksamkeit bei 100 und 200 mg/kg Körpergewicht. Ebenfalls zeigt sich der für Pflanzenextrakte der typische Effekt eines optimalen Dosisfensters, da die höhere Dosierung keine verbesserte Wirksamkeit zeigt.

### Rückstandfraktionen

Abgetrennte Rückstandsfraktion von erfindungsgemäßem Extrakt, Charge UB 2007-37-1, gemäß Beispiel 3

| | |
|---|---|
| Auszugsmittel | EtOH 35% V/V |
| Aufreinigung | Ethanolisches Eluat von Adsorbersäule mit XAD-4 Harz |
| DEV nativ | 4,2 : 1 |
| Gehalt Gesamt-Valerensäuren | 0,73 % |
| Gehalt Valepotriate | 0,21 % |
| Anxiolytische Eigenschaften im EPM-Modell (weibl. NMRI-Mäuse nüchtern, n = 10, 60 min. nach oraler Gabe), 250 mq/kq KG | |
| %-Anteil Verweildauer offene Arme | + 12 % |
| %-Anteil Eintritte offene Arme | + 11 % |
| Wertung | Niedrigere Werte gegenüber Kontrollgruppe, indifferent bis gegensätzliche Wirkung (anxiogen) |

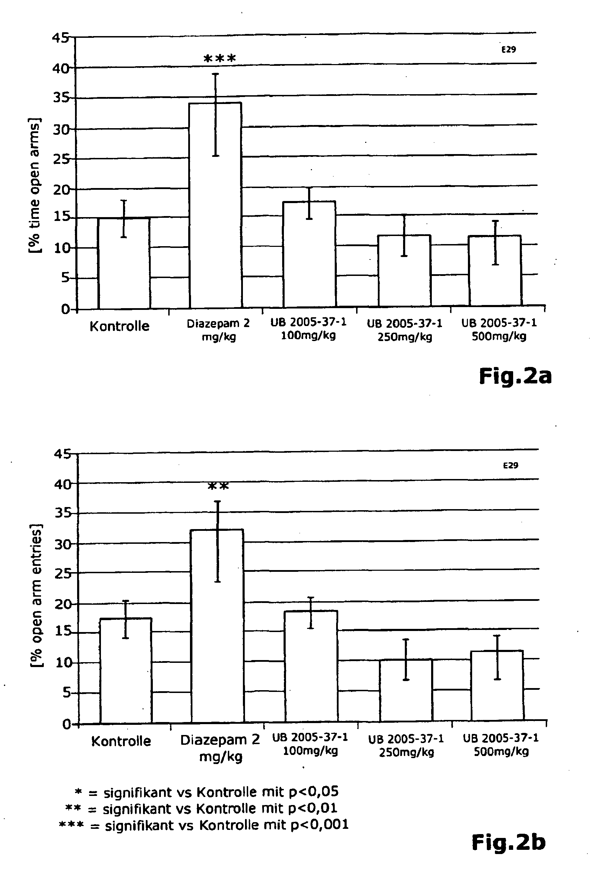

Zusätzlich wurde die Rückstandsfraktion vom Adsorber (Beispiel 3) untersucht. Figur 2a/b zeigen die Untersuchung dieser Substanz im EPM.

Figur 2a) zeigt dabei die Aufenthaltszeit auf dem offenen Arm.

Figur 2b) zeigt die Anzahl der Eintritte in den offenen Arme.

Es zeigt sich, dass die im Adsorber gefundene Substanz mit einem hohen Gehalt an Valerensäuren sogar eher einen anxiogenen Effekt hat. Durch das erfindungsgemäße Verfahren werden der Anxiolyse antagonistisch wirkende Substanzen gezielt abgereichert.

Figur 3 zeigt die dünnschichtchromatographische Analyse der Fraktionen.

Bahn 3: erhaltener Rohextrakt gemäß Beispiel 1.

Bahn 2: erfindungsgemäßer Extrakt gemäß Beispiel 2a).

Bahn 1: vom Adsorber eluierbare Rückstandsfraktion gemäß Beispiel 3.

### Beispiel 7: Testung auf Sedierung

Die sedierende Wirkung wurde durch Untersuchung der spontanen Motilität geprüft. Hierzu wurden NMRI-Mäuse (Charles River, Sulzfeld, n = 8 pro Gruppe) mittels Infrarottechnik unmittelbar nach Gabe der Prüflösung auf eine sedierende Wirkung getestet, in dem die Bewegungsaktivität gemessen wurde. Hierzu werden in einem Käfig Linienübertritte innerhalb eines 10 Minuten Intervalls gemessen. Im Vergleich zur Kontrolle wies der erfindungsgemäße Extrakt keine sedierende Wirkung auf.

### Beispiel 8: Testung auf Narkosedauer

In einem weiteren Test wurde die etherinduzierte Narkosedauer an weiblichen NMRI-Mäusen (Charles River, Sulzfeld, n = 8) untersucht. Es konnte keine Verlängerung der Narkosedauer beobachtet werden; dies zeigt ebenfalls, dass die erfindungsgemäßen Extrakzubereitungen keine sedierende Wirkung haben.

### Beispiel 9: Untersuchung der antidepressiven Wirkung

Eine spezifischere Testung auf antidepressive Aktivität bietet der von Porsolt entwickelte Forced Swimming Test (FST) (Porsolt et al., 1978. Behavioural despair in rats: a new model sensitive to antidepressant treatments, Eur J Pharmacol 47, 379-391).

Die Versuchstiere (Ratten) werden in einem sogenannten "Schwimmtest", mit einer Stresssituation konfrontiert, der sie aus eigener Kraft nicht entkommen können. Dazu werden Ratten zunächst in einem Vorversuch (Konditionierung), einen Tag vor dem eigentlichen Test, für die Dauer von 15 Minuten in einem mit Wasser gefüllten Glaszylinder belassen. Die Füllhöhe ist auf das Körpergewicht der Tiere abgestimmt, so dass sich jedes Tier mit Pfoten und Schwanz abstützen und mit minimalen Schwimmbewegungen die Nase über Wasser halten kann. Die Tiere lernen, nach einer länger andauernden Phase heftiger Schwimm- und Tauchbewegungen, dass eine Befreiung aus der Situation nicht möglich ist und nehmen eine charakteristische, nahezu bewegungslose, gekrümmte Körperhaltung mit weitgehend geschlossener Lidspalte an ("despair behavior"). 24 Stunden nach diesem Vorversuch erfolgt die Testung mit Baldrian-Extrakt, in dem die Ratten erneut in die Wasserzylinder eingebracht werden und über einen Zeitraum von 5 Minuten die Dauer der Immobilität kumulativ erfasst wird.

Für die Testung der Baldrianzubereitungen an männlichen CD-Ratten wurde 16 Tage lang vorbehandelt, eingesetzt wurden die Zubereitung gemäß Beispiel 2b). Fünf Stunden nach der letzten Behandlung wurden die Ratten in 40 cm hohe, durchsichtige Plexiglaszylinder mit einem Durchmesser von 18 cm eingesetzt. Die Zylinder wurden vor jedem Versuchsdurchgang mit 25 ± 1°C warmen Wasser befüllt, die Füllhöhe betrug in Abhängigkeit vom Rattengewicht 17-20 cm. Der Versuch wurde mittels Videokamera aufgezeichnet und die Immobilitätsdauer der Tiere später am Monitor manuell erfasst.

Antidepressiva wie Diazepam bewirken eine Verkürzung der Immobilitätsdauer, die Tiere verbleiben eine längere Zeit in der aktiven Schwimmphase.

In diesem Test verkürzte 1 mg/kg Diazepam die Immobilitätsdauer um 28% gegenüber der Kontrollgruppe. Für einen Primärextrakt aus Baldrianwurzeln mit EtOH 35% (UB 2004-18, Beispiel 5) wurde bei einer Dosierung von 125 mg/kg keine Veränderung gegenüber der Kontrollgruppe festgestellt. Demgegenüber erreichte der erfindungsgemäße Baldrianextrakt nach Aufreinigung über hydrophobe Adsorber (UB 2005-78-2, Beispiel 2b)) bei gleicher Dosierung von 125 mg/kg eine Reduzierung der Immobilitätsdauer um 36%, die gegenüber der Kontrollgruppe statistisch signifikant war.

Ferner ergibt sich ein statistisch hoch signifikanter Unterschied zwischen der Konzentration 12,5 mg und der 10fachen Dosierung von 125 mg.

### Beispiel 10: Herstellung Dickextrakt

10,5 kg von Radix Valerianae offic. (Valerensäuren 0,23%) wurden in einen Extrakteur eingefüllt und für 3 h mit Heißwasserdampf von unten nach oben behandelt. Dabei wurden flüchtige Inhaltsstoffe aus dem Extrakteur abgeführt und kondensiert. Das wässrige Kondensat innerhalb und außerhalb des Extrakteurs wurde verworfen. Anschließend wurde die so vorbehandelte Droge mit 30% v/v Ethanol im Verhältnis 1:14 versetzt und bei 40°C im Holstein-Kappert-Extrakteur erschöpfend perkoliert. Das Eluat wurde von der Droge abgelassen und über einen 250µm Siebbeutel-Durchlass von Drogenresten befreit. Das Perkolat wurde einem Plattenverdampfer eingedampft und durch Wasserzugabe lösemittelfrei gefahren.

Als Ausbeute resultierten 4,9 kg Dickextrakt mit einem Trockensubstanzanteil von 66,0%. Berechnet auf den nativen Trockenextrakt sind enthalten < 0,02% Valepotriate, 0,015% iso-Valeriansäure, 0,23% Gesamt-Valerensäuren, hiervon 0,11% Valerensäure, 0,09% Acetoxyvalerensäure und 0,03% Hydroxyvalerensäure.

### Beispiel 11: erfindungsgemäßes Extrakt

Ein Anteil von 606 g Extrakt von Beispiel 10, entsprechend 400 g Trockenextraktequivalent wurde mit demineralisiertem Wasser auf 10% Trockensubstanzanteil verdünnt und 30 Minuten lang unter Rühren homogenisiert. Es resultierte eine dunkle, homogene Lösung.

Diese wurde auf ein hydrophobes Adsorberharz (XAD-4) in einer Glassäule gegeben, die mit ca. 41 feuchtem Harz, entsprechend ca. 1600 g Trockenharz, gefüllt ist. Mit einer Geschwindigkeit von 3 Bettvolumina der Harzes pro Stunde erfolgte der Reinigungsschritt. Nach Reinigung der Ausgangsextraktlösung konnten noch 83% der Ausgangsmenge festgestellt werden.

Diese Lösung wurde zum Dickextrakt beigedampft und mit dem Trockenhilfsstoff Maltodextrin im Verhältnis 80% nativ : 20% Maltodextrin per Sprühtrocknung in eine Trockenextraktzubereitung überführt (UB 2006-66).

Diese geruchsarme Baldrianextraktzubereitung hatte einen Trocknungsverlust von 4,0% und enthielt: < 0,02% Valepotriate, 0,008% iso-Valeriansäure, 0,113% Gesamt-Valerensäuren, hiervon 0,07% Valerensäure, 0,04% Acetoxyvalerensäure und 0,003% Hydroxyvalerensäure.

Die Testung im EPM-Modell auf anxiolytische Eigenschaften ergab bei 100 mg Dosierung / kg Körpergewicht weiblicher NMRI-Ratten, 60 min nach oraler Gabe: Steigerung der Zeit auf den offenen Armen um 30%; Steigerung der Anzahl der Eintritte auf die offenen Arme um 25%, im Vergleich zur Kontrolle.

### Beispiel 12: Herstellung einer pharmazeutischen Zusammensetzung

Der erfindungsgemäße Extrakt wird mit nachfolgender Rezeptur einer Direkttablettierung unterworfen.

| | |
|---|---|
| 300 mg | erfindungsgemäßer Baldrian-Trockenextrakt |
| 160 mg | mikrokristalline Cellulose |
| 25 mg | Natriumcarboxymethylcellulose |
| 10 mg | hochdisperses Siliziumdioxid |
| 5 mg | Magnesiumstearat |

Die resultierende Tablette wird zur Minderung des typischen Baldriangeruches mit einem Überzug an Eudragit E100 gecoated.

### Ergebnisse der Experimente

Der erfindungsgemäß Extrakt kombiniert somit eine antidepressive und anxiolytische Wirksamkeit ohne eine allgemein sedierende Wirkung aufweisen.

## Patentansprüche

1. Verfahren zur Herstellung einer Baldrian-Extraktzubereitung umfassend die Schritte der
a) Extraktion von Radix Valerianae officinalis mit alkoholisch-wässrigen Extraktionsmitteln, um einen Rohextrakt zu erhalten, wobei das alkoholisch-wässrige Extraktionsmittel zwischen 10 und 50 Vol.-% Alkohol enthält;
b) zumindest teilweises Entfernen des alkoholischen Anteils aus dem Rohextrakt, um einen Dickextrakt zu erhalten;
c) Inkontaktbringen des Dickextraktes mit einem hydrophoben Adsorptionsmittel;
d) Abtrennen des hydrophoben Adsorptionsmittel, um einen gereinigten Extrakt zu erhalten,
e) Überführung des gereinigten Extraktes in eine Baldrian-Extraktzubereitung mit pharmazeutisch üblichen Hilfsstoffen.

2. Verfahren nach Anspruch 1, **dadurch gekennzeichnet, dass** der Alkohol ausgewählt wird aus Methanol, Ethanol, Isopropanol und Mischungen davon.

3. Verfahren nach Anspruch 1 oder 2, **dadurch gekennzeichnet, dass** der Alkohol im Rohextrakt durch Verdampfen entfernt wird.

4. Verfahren nach mindestens einem der Ansprüche 1 bis 3, **dadurch gekennzeichnet, dass** der hydrophobe Adsorber ausgewählt wird aus der Gruppe Divinylbenzolcopolymeren, aliphatischen Esterpolymeren und Formophenolpolymeren.

5. Verfahren nach mindestens einem der Ansprüche 1 bis 4, **dadurch gekennzeichnet, dass** die Baldrian-Extraktzubereitung in eine Trockenextraktzubereitung zusammen mit Trockenhilfsstoffen überführt wird.

6. Baldrian-Extraktzubereitung erhältlich nach einem Verfahren gemäß einem der Ansprüche 1 bis 5.

7. Baldrian-Extraktzubereitung nach Anspruch 6 **gekennzeichnet durch** einen Gehalt von maximal
- Gesamt-Valerensäuren ≤ 0,18 Gew.-%, hiervon
• Acetoxyvalerensäuren ≤ 0,1%,
• Hydroxyvalerensäuren < 0,01%,
- Valepotriaten ≤ 0,1 Gew.-%, jeweils bezogen auf den nativen Trockenextrakt.

8. Baldrian-Extraktzubereitung nach mindestens einem der Ansprüche 6 oder 7, **dadurch gekennzeichnet, dass** das Droge/Extrakt/Verhältnis zwischen 3:1 und 10:1 liegt.

9. Arzneimittel oder Nahrungsergänzungsmittel enthaltend eine Baldrian-Extraktzubereitung nach mindestens einem der Ansprüche 6 bis 8.

10. Verwendung einer Baldrian-Extraktzubereitung nach mindestens einem der Ansprüche 6 bis 8 zur Herstellung eines Arzneimittels oder Nahrungsergänzungsmittels zur Angsthemmung und Angstlösung (Anxiolytikum).

11. Verwendung einer Baldrian-Extraktzubereitung nach mindestens einem der Ansprüche 6 bis 8 zur Herstellung eines Arzneimittels oder Nahrungsergänzungsmittels zur Minderung von Depressionen oder depressiven Stimmungen (Antidepressivum).

## Claims

1. A process for preparing a valerian extract formulation comprising the steps of:
a) extracting radix valeriana officinalis with alcoholic-aqueous extractants to obtain a raw extract, said alcoholic-aqueous extractant containing from 10 to 50% by volume of alcohol;
b) at least partially removing the alcoholic fraction from the raw extract to obtain a viscous extract;
c) contacting the viscous extract with a hydrophobic adsorbing agent;
d) separating the hydrophobic adsorbing agent to obtain a purified extract;
e) converting the purified extract to a valerian extract formulation using pharmaceutically acceptable auxiliary agents.

2. The process according to claim 1, **characterized in that** said alcohol is selected from methanol, ethanol, isopropanol and mixtures thereof.

3. The process according to claim 1 or 2, **characterized in that** said alcohol in the raw extract is removed by evaporation.

4. The process according to at least one of claims 1 to 3, **characterized in that** said hydrophobic adsorber is selected from the group consisting of divinylbenzene copolymers, aliphatic ester polymers and formophenol polymers.

5. The process according to at least one of claims 1 to 4, **characterized in that** said valerian extract formulation is converted to a dry extract formulation together with drying auxiliaries.

6. A valerian extract formulation obtainable by a process according to any of claims 1 to 5.

7. The valerian extract formulation according to claim 6, **characterized by** a content of at most:
- total valerenic acids ≤ 0.18% by weight, among which
• acetoxyvalerenic acids < 0.1%;
• hydroxyvalerenic acids < 0.01%;
- valepotriates ≤ 0.1% by weight, respectively based on the native dry extract.

8. The valerian extract formulation according to at least one of claims 6 or 7, **characterized in that** its herb-to-extract ratio is from 3:1 to 10: 1.

9. A medicament or food supplement containing a valerian extract formulation according to at least one of claims 6 to 8.

10. Use of a valerian extract formulation according to at least one of claims 6 to 8 for preparing a medicament or food supplement for inhibiting or releasing anxiety (anxiolytic).

11. Use of a valerian extract formulation according to at least one of claims 6 to 8 for preparing a medicament or food supplement for reducing depressions or depressive moods (antidepressive).

## Revendications

1. Procédé de production d'une préparation d'extrait de valériane, comprenant les étapes suivantes :
a) extraction de Radix Valerianae officinalis avec des agents d'extraction hydro-alcooliques pour obtenir un extrait brut, l'agent d'extraction hydro-alcoolique contenant entre 10 et 50 %-vol. d'alcool ;
b) élimination au moins partielle de la partie alcoolique à partir de l'extrait brut afin d'obtenir un extrait épais ;
c) mise en contact de l'extrait épais avec un agent d'adsorption hydrophobe,
d) séparation de l'agent d'adsorption hydrophobe pour obtenir un extrait purifié,
e) transformation de l'extrait purifié en une préparation d'extrait de valériane avec des adjuvants couramment utilisés en pharmacie.

2. Procédé selon la revendication 1, **caractérisé en ce que** l'alcool est sélectionné parmi le méthanol, l'éthanol, l'isopropanol et des mélanges de ceux-ci.

3. Procédé selon la revendication 1 ou 2, **caractérisé en ce que** l'alcool dans l'extrait brut est enlevé par évaporation.

4. Procédé selon au moins une des revendications 1 à 3, **caractérisé en ce que** l'adsorbeur hydrophobe est sélectionné dans le groupe copolymères de divinylbenzène, polymères d'ester aliphatiques et polymères de formophénol.

5. Procédé selon au moins une des revendications 1 à 4, **caractérisé en ce que** la préparation d'extrait de valériane est transformée en une préparation d'extrait sec ensemble avec des adjuvants secs.

6. Préparation d'extrait de valériane pouvant être obtenue selon un procédé selon une des revendications 1 à 5.

7. Préparation d'extrait de valériane selon la revendications 6, **caractérisée par** une teneur au maximum
- d'acides valéréniques totaux ≤ 0,18 %-poids, dont
■ acides acétoxyvaléréniques < 0,1 %,
■ acides hydroxyvaléréniques < 0,01 %,
- valépotriates ≤ 0,1 %-poids, respectivement rapportés à l'extrait sec natif.

8. Préparation d'extrait de valériane selon au moins une des revendications 6 ou 7, **caractérisée en ce que** le rapport drogue/extrait est entre 3:1 et 10:1.

9. Médicament ou complément alimentaire contenant une préparation d'extrait de valériane selon une des revendications 6 à 8.

10. Utilisation d'une préparation d'extrait de valériane selon au moins une des revendications 6 à 8 pour la production d'un médicament ou d'un complément alimentaire destiné à atténuer et traiter l'anxiété (anxiolytique).

11. Utilisation d'une préparation d'extrait de valériane selon au moins une des revendications 6 à 8 pour la production d'un médicament ou d'un complément alimentaire destiné à lutter contre les dépressions ou les états dépressifs (antidépresseurs).
